# EUROPEAN PATENT APPLICATION

(11) **EP 4 043 871 A1**
(43) Date of publication of application: **17.08.2022**
(21) Application number: 21157232.6
(22) Date of filing: 15.02.2021
(51) Int. Cl.: G01N 23/20, C07D 213/06, C07D 401/14

(54) **SELECTION METHOD FOR CONDITIONS TO INTRODUCE A GUEST ANALYTE COMPOUND INTO CRYSTALLINE POLYNUCLEAR METAL COMPLEX FOR USE OF SUCH CRYSTALLINE POLYNUCLEAR METAL COMPLEX INCLUDING A GUEST COMPOUND ANALYTE IN A METHOD FOR DETERMINING MOLECULAR STRUCTURE OF THE GUEST COMPOUND ANALYTE**

(71) Applicant: Merck Patent GmbH, 64293 Darmstadt (DE)
(72) Inventor: VON ESSEN, Carolina, 64293 Darmstadt (DE); ROSENBERGER, Lara, 64293 Darmstadt (DE)
(74) Representative: Merck Serono S.A. Intellectual Property

(57) **Abstract**

The present invention is directed to methods for selecting (solution) conditions to introduce an analyte guest compound into a crystalline polynuclear metal complex. The selected (solution) conditions can be used in a method for introducing an analyte guest compound into a crystalline polynuclear metal complex to form a crystal structure analysis sample. The molecular structure of the analyte guest compound can be determined by X-ray crystallography using the crystal structure analysis sample obtained with such method.

## Description

### Field of the Invention

The present invention relates to crystalline polynuclear metal complexes which include a guest compound analyte. Such crystalline polynuclear complexes including a guest analyte compound are useful in crystal structure analysis methods to determine the molecular structure of such guest compound analyte present in the such crystalline polynuclear metal complex. In the present invention selection methods are described to determine the conditions for introducing such guest compound analyte into the crystalline polynuclear metal complex. The present invention also relates to the use of such crystalline polynuclear complexes which include a guest compound analyte so obtained in methods for determining the molecular structure of the guest compound analyte through crystal structure analysis.

### Background

X-ray single crystal structure analysis has been known as a method for determining the molecular structure of an organic compound. The molecular structure of an organic compound can be accurately determined using X-ray single crystal structure analysis when it is possible to prepare a high-quality single crystal.

However, when the amount of organic compound is very small, and it is impossible to obtain a sufficient amount of single crystal, it is difficult to employ X-ray single crystal structure analysis for determining the molecular structure of the organic compound. It is difficult to prepare a single crystal when the organic compound for which the molecular structure is to be determined is liquid at about room temperature (i.e., when the melting point of the organic compound is equal to or lower than room temperature). This difficulty may be for example as a result of the inability of the compound in forming crystals at room temperature. Also, the difficulty can simply be that it is extremely difficult to obtain sufficient quantities of appropriate purity of the compound analyte. The use of a crystalline polynuclear complex, which is porous, acts as a framework to support the compound analyte in an ordered manner.

The use of crystalline polynuclear metal complexes that can incorporate a guest compound analyte in an ordered manner has enabled the use of X-ray single crystal structure analysis even in instances wherein it is difficult to obtain a sufficient amount of a single crystal of the analyte. Such method of determining the molecular structure of the analyte compound is known as the crystalline sponge method wherein the crystalline polynuclear metal complex is considered the crystalline sponge. This emerging technology in X-ray crystallography makes it possible to obtain the crystal structure of non-crystalline (or hard-to-crystallize) compounds, without crystallization, on a microgram scale. The use of crystalline polynuclear metal complexes or crystalline sponges for facilitated structure elucidation of small molecules is described in for example WO 2014/038220 and WO 2016/143872 and in for example Y. Inokuma et al., Nature 2013, 495, 461-466 (Corrigendum: Nature 2013, 501) and M. Hoshino, et al., IUCrJ, 2016, 3, 139-151. This method uses a porous crystalline polynuclear metal complex, for example [(ZnX₂)₃•(tpt)₂]ₙ (wherein X = CI or I; and tpt = 2,4,6-tris(4-pyridyl)-1,3,5-triazine) as a crystalline sponge that can absorb small to medium sized organic molecules, orient them uniformly in their pores, and make them observable by conventional single crystal X-ray crystallography.

However, poor affinity of an analyte guest molecule into the crystalline polynuclear metal complex framework could result in insufficient amounts of such guest molecule to be in an ordered configuration within the framework compromising the structure elucidation through X-ray crystallography. To obtain sufficient amounts of the analyte guest compound into the crystalline polynuclear metal complex, it is important to select an individual optimization of "soaking conditions" for the analyte. The term "soaking conditions" refers to the process conditions for introducing such analyte into the crystalline polynuclear metal complex. It may take weeks to adjust the different soaking combinations of type crystalline polynuclear metal complex, organic solvent, soaking duration and temperature to achieve successful results. To examine successful analyte absorption and a regular order of guest molecules by intermolecular, noncovalent interactions within the "Crystalline Sponge" (crystalline polynuclear metal complex) framework, time-consuming XRD measurements may be necessary.

To determine the affinity to the crystalline polynuclear metal complexes prior to X-ray diffraction experimentation and lengthy purification and separation processes, Wada et. al. (Crystalline-Sponge-Based Structural Analysis of Crude Natural Product Extract; Angew. Chem. Int. Ed. Engl. 57, 3671-3675 (2018)) described a method to overcome this problem of unpredictable affinities of analyte guest compounds to the crystalline sponge framework. The method describes the soaking of crystalline sponges with a complex mixture of compounds. After the soaking, the soaked compounds were re-extracted from the crystalline sponges and were analyzed through HPLC-UV. The fractions found in the re-extracted solution were compared to the fractions found initial complex mixture, to determine whether a compound has or has no affinity to the sponge and to prioritize separation, purification and XRD on those compounds which exhibit a good affinity to the crystalline sponge.

The method described by Wada et al. is intended to demonstrate the application of a prior affinity screening for a compound mixture using one soaking condition (using [(Znl₂)₃ - (tpt)₂·x(cyclohexane)]ₙ; at 50°C; for 6 days). In addition, such re-extraction process to recover the analyte from the crystalline sponge pores takes required 4 days. As such it is not suitable as a quick test for single compounds/mixtures under various conditions with a single substance to determine if a crystalline sponge analyte has affinity. More importantly such method is inadequate to determine the optimal conditions (crystalline sponge type, solvent, temperature) would be the best to provide sufficient inclusion of the analyte guest compound into the crystalline sponge for obtaining successful crystallographic results by X-ray diffraction measurements (XRD).

Furthermore, the method by Wada et al. potentially results in false positive outcomes due to substance (analyte guest compound) amounts adsorbed on the crystal surfaces (e.g. of the crystalline sponge crystals) but which are unable to penetrate the pores of the crystalline sponge and which adsorbed substance are not accessible to XRD. Finally, the method described by Wada et. al. uses HPLC-UV which is challenging for very low amounts of analyte (like e.g. in the case of identification of metabolites and impurities).

The method of the present invention provides a solution to the above described shortcomings. The present invention provides a selection method including parallel screening of for the best soaking conditions while analyzing the result after washing the guest compound crystalline sponge complex with an appropriate washing fluid.

### Summary of the Invention

The current invention provides a solution to the above described problems in screening for the optimal soaking conditions to obtaining sufficient analyte guest compounds within the crystalline polynuclear metal complexes. In the selection method of the present invention the affinity screening includes multiple different soaking conditions (type of crystalline sponge material, solvent, temperature) which are used in parallel. This allows to not only determine whether the guest analyte compounds have an affinity to the crystalline sponges for inclusion in its porous framework, but also directly identify the best optimal conditions. The false positive signal from analyte guest compounds that adsorb to the surface of the crystalline sponge is minimized by washing the sponges with a washing fluid medium. The lengthy re-extraction process is substituted by dissolution of the sponges that liberates the soaked components much quicker (12-24 hours instead of 4 days). Finally, the qualitative and quantitative analysis of analyte guest compounds introduced into the crystalline sponge during soaking is conducted with more sensitive and faster methods such as for example UPLC/-MS/-MS. With the soaking conditions so selected a guest analyte compound can be successfully introduced into the crystalline sponge material in sufficient amounts to allow for taking useful X-ray diffraction measurements in using X-ray crystallography for the structure elucidation of the analyte guest compound.

In one embodiment the present invention provides a method to select conditions for inclusion of an analyte in a crystalline polynuclear metal complex for use in single crystal X ray crystallography, wherein the crystalline polynuclear metal complex represented by [[M(X)₂]₃(L)₂]ₙ, wherein M is a metal ion, X is a monovalent anion, L is a tridentate ligand represented by a formula (1), 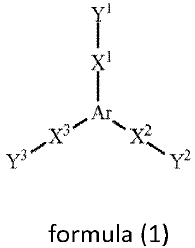 wherein Ar is a substituted or unsubstituted trivalent aromatic group, X¹ to X³ are independently a divalent organic group, or a single bond that directly bind Ar and Y¹, Y², or Y³, and Y¹ to Y³ are independently a monovalent organic group having a coordinating moiety, and n is an arbitrary natural number, the method comprising:
a) contacting a plurality of samples of the analyte with the crystalline polynuclear metal complex under a plurality of solution conditions to obtain a plurality of sample analyte polynuclear metal complexes in solution,
b) evaporating the solvent from each sample analyte polynuclear metal complex,
c) determine the amount of analyte included in each sample analyte polynuclear metal complex, and
d) selecting the solution condition resulting in the highest amount of analyte included in the sample analyte polynuclear metal complex.

In another embodiment the steps a) to c) are carried out in parallel. In yet another embodiment the step c) includes a washing step removing sample analyte which is adsorbed onto the crystalline polynuclear metal complex but has not been introduced within its framework and thereafter dissolving the sample analyte polynuclear metal complex followed by the determination of the amount of sample analyte introduced into the crystalline polynuclear metal complex.

In yet another embodiment there is provided a method to select conditions for inclusion of an analyte in a crystalline polynuclear metal complex for use in single crystal X-ray crystallography, wherein the crystalline polynuclear metal complex represented by [[M(X)₂]₃(L)₂]ₙ, wherein M is a metal ion, X is a monovalent anion, L is a tridentate ligand represented by a formula (1), 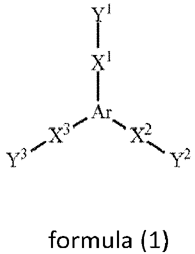 wherein Ar is a substituted or unsubstituted trivalent aromatic group, X¹ to X³ are independently a divalent organic group, or a single bond that directly bind Ar and Y¹, Y², or Y³, and Y¹ to Y³ are independently a monovalent organic group having a coordinating moiety, and n is an arbitrary natural number, the method comprising:
a) contacting a plurality of samples of the analyte with the crystalline polynuclear metal complex under a plurality of solution conditions to obtain a plurality of sample analyte polynuclear metal complexes in solution,
b) evaporating the solvent from each sample analyte polynuclear metal complex,
c) determine the amount of analyte included in each sample analyte polynuclear metal complex comprising the steps of
   i. washing the sample analyte polynuclear metal complex obtained in step b),
   ii. dissolving the sample analyte polynuclear metal complex obtained in the previous step, and
   iii. determine the amount of sample analyte introduced into the crystalline polynuclear metal complex using a quantitative analytical method, and
d) selecting the solution condition resulting in the highest amount of analyte included in the sample analyte polynuclear metal complex.

In yet another embodiment there is provided a method of producing a crystal structure analysis sample, comprising selecting the solution conditions for introducing an analyte into crystalline polynuclear metal complex and contacting the analyte and crystalline polynuclear metal complex at the solution conditions selected in the previous step, wherein the crystalline metal complex is represented by [[M(X)₂]₃(L)₂]ₙ, wherein M is a metal ion, X is a monovalent anion, L is a tridentate ligand represented by a formula (1), 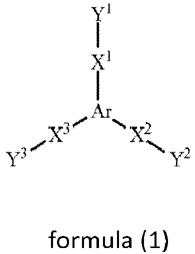 wherein Ar is a substituted or unsubstituted trivalent aromatic group, X¹ to X³ are independently a divalent organic group, or a single bond that directly bind Ar and Y¹, Y², or Y³, and Y¹ to Y³ are independently a monovalent organic group having a coordinating moiety, and n is an arbitrary natural number to arrange molecules of the analyte in the pores and/or voids of the crystalline polynuclear metal complex in an ordered manner. This method comprises introducing an analyte into a crystalline polynuclear metal complex for use in single crystal X ray crystallography, wherein the crystalline polynuclear metal complex represented by [[M(X)₂]₃(L)₂]ₙ, wherein M is a metal ion, X is a monovalent anion, L is a tridentate ligand represented by a formula (1), 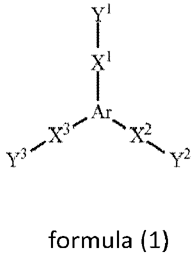 wherein Ar is a substituted or unsubstituted trivalent aromatic group, X¹ to X³ are independently a divalent organic group, or a single bond that directly bind Ar and Y¹, Y², or Y³, and Y¹ to Y³ are independently a monovalent organic group having a coordinating moiety, and n is an arbitrary natural number, the method comprising:
a. selecting the solution conditions for introducing the analyte into the crystalline polynuclear metal complex using the method according to any of the preceding claims, and
b. contacting the analyte and crystalline polynuclear metal complex at the solution conditions selected in step a) to obtain an analyte crystalline polynuclear metal complex.

In another embodiment of the present invention there is provided a method for determining a molecular structure of an analysis target compound, comprising performing crystal structure analysis using a crystal structure analysis sample obtained by a method for producing the crystal structure analysis sample obtained under conditions selected using a method of the present invention of selecting the optimal solution conditions for introducing an analyte compound into a crystalline polynuclear metal complex.

### Brief Description of the Drawings

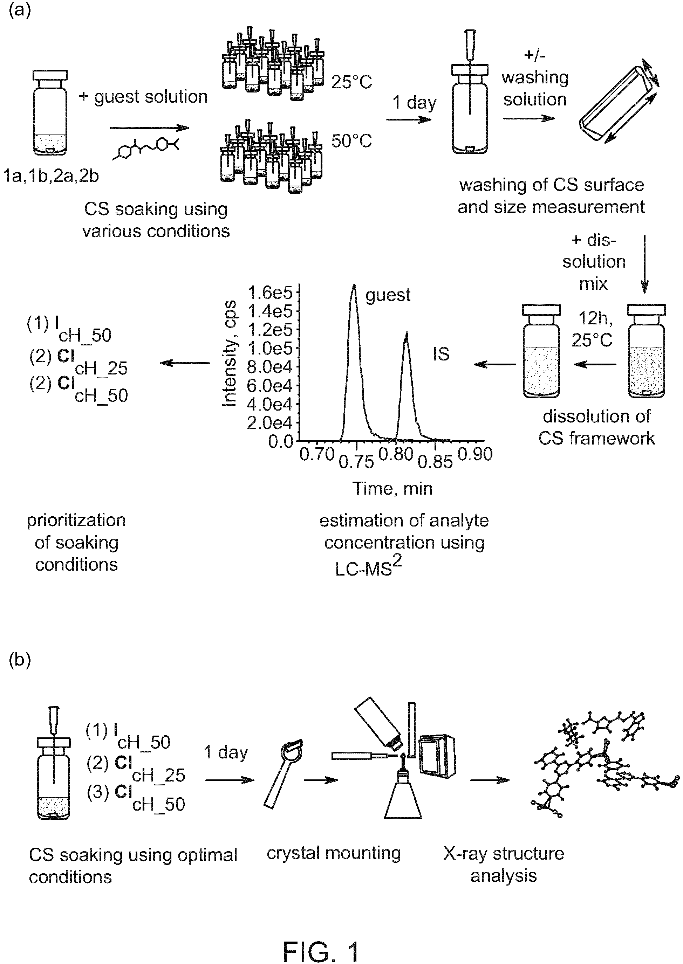

Figure 1, shows a schematic representation of the selection method including a complete workflow of the affinity screening from optimization of soaking conditions to the final generation of crystallographic data.

### Detailed Description of the Invention

Methods for selecting optimal (solution) conditions for introducing an analyte guest compound into a crystalline polynuclear metal complex, and the use of such selected method in introducing such analyte guest compound into a crystalline polynuclear metal complex (crystalline sponge) to obtain an analyte crystalline sponge complex for use in crystal X-ray crystallography for the elucidation of the molecular structure of an analyte guest compound are described in detail below. These analyte guest compound crystalline polynuclear metal complexes can be used in methods for the structure elucidation of the guest analytes using the crystalline sponge technology. The crystalline sponge technology makes use of crystalline polynuclear metal complexes as a crystalline molecular sponge for an analyte to arrange such analyte in an ordered manner. A complex so formed comprising the analyte in a crystalline polynuclear metal complex allows for X-ray crystallographic analysis of the analyte.

The crystalline polynuclear metal complexes (crystalline sponges) used in connection with the embodiments of the invention have a three-dimensional network structure that includes a ligand having coordinating moieties, and a metal ion that serves as a center metal. The term "three-dimensional network structure" used herein refers to a network-like structure in which a structural unit formed by a ligand (i.e., a ligand having two or more coordinating moieties and an additional monodentate ligand) and a metal ion that is bonded to the ligand is repeatedly arranged three-dimensionally.

The three-dimensional network structure results in the crystalline polynuclear metal complex to have a porous structure. Such porous structure enables the capturing of an analyte guest compound within the porous structure of a crystalline polynuclear metal complex. As such the crystalline polynuclear metal complex acts as a "sponge" for the analyte guest compound. Introduction of such analyte guest compound in a crystalline complex of the present invention allows for the organized arrangement of the analyte guest compound within the complex molecule. This organized arrangement of the analyte guest compound enables the use of single crystal X-ray crystallography to elucidate the molecular structure of the analyte guest compound. Therefore, the single crystal X-ray crystallography method using such crystalline polynuclear metal complexes is often referred to as the Crystalline Sponge (CS) X-ray crystallography method.

The use of the crystalline sponge technology relies on the successful introduction of the analyte guest compound into the crystalline polynuclear complex ("crystalline sponge"). For each analyte guest compound, the conditions for successful introduction into the crystalline sponge material would need to be optimized and selection of the proper conditions can be time consuming or shows absorption to the surface of the crystalline sponge. Only analyte guest compound introduced into the crystalline sponge are being arranged in an ordered manner and X-ray diffraction measurements would allow only structure elucidation for such introduced analyte guest compound. Current methods often result in selection of non-optimal (solution) conditions for introducing the analyte guest compound into the crystalline sponge while at the same time being very time consuming. It is important that sufficient amounts of the analyte guest compound are being introduced in the crystalline sponge material using a selected (solution) condition ("soaking conditions").

The present invention described herein is directed to such selection methods for screening for the (solution) conditions ("soaking conditions") for introducing a sufficient amount of the analyte guest compound into the crystalline polynuclear metal complex. The method included the screening of a plurality of soaking conditions in parallel for selecting the optimal soaking conditions. In addition, in the method of the present invention the problems with previously used methods are overcome with a method including a washing step of the complex formed from the analyte guest compound and the crystalline polynuclear metal complex. Furthermore, dissolving such complex formed from a analyte guest compound being introduced into the crystalline polynuclear metal complex would ameliorate the previously observed problems, preferably even more so when accompanied by the aforementioned washing step. As a result of the selection method of the present invention, the optimal soaking conditions can be selected for a particular analyte guest compound more rapidly, without extensive experimentation with process of long duration. Moreover, the selection method of the present invention would allow for screening for optimal soaking conditions significantly reducing the amount of false positive outcomes of such screening method allowing the selection of the optimal soaking conditions for introducing the analyte guest compound into the crystalline sponge with relative ease.

One selection that needs to be made when attempting to introduce an analyte guest compound into a crystalline polynuclear metal complex (crystalline sponge) is the type of such crystalline sponge which is optimal for the particular analyte guest compound. The crystalline polynuclear metal complex for use in the present invention includes a coordinating metal complex and is represented by [[M(X)₂]₃(L)₂]ₙ wherein
M is a metal ion,
X is a monovalent anion,
L is a tridentate ligand represented by formula (1),

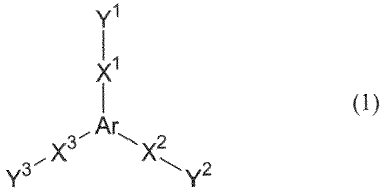

wherein Ar is a substituted or unsubstituted trivalent aromatic group, X¹ to X³ are independently a divalent organic group, or a single bond that directly bonds Ar and Y¹, Y², or Y³, and Y¹ to Y³ are independently a monovalent organic group having a coordinating moiety,
n is an arbitrary natural number, and
p is an arbitrary number.

The tridentate aromatic ligand Ar in the formula (1) is a trivalent aromatic group. The number of carbon atoms of Ar is normally 3 to 22, preferably 3 to 13, and more preferably 3 to 6. Examples of Ar include a trivalent aromatic group having a monocyclic structure that consists of one 6-membered aromatic ring, and a trivalent aromatic group having a fused ring structure in which three 6-membered aromatic rings are fused.

Examples of the trivalent aromatic group having a monocyclic structure that consists of one 6-membered aromatic ring include the groups respectively represented by the following formulas (2a) to (2d). Examples of the trivalent aromatic group having a fused ring structure in which three 6-membered aromatic rings are fused, include the group represented by the following formula (2e). It is noted that"*" in the formulas (2a) to (2e) indicates the positions at which X¹ to X³ are bonded.

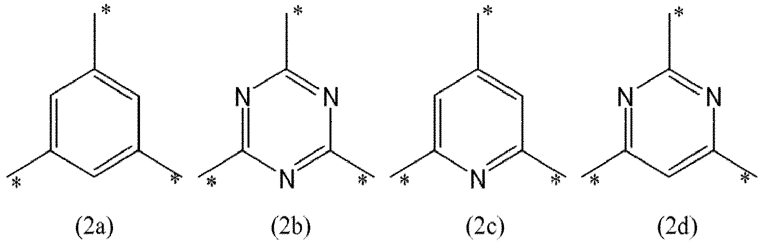

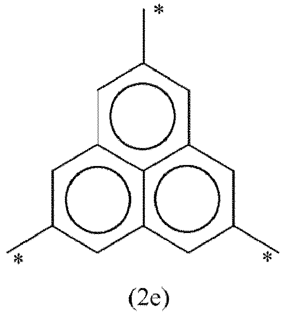

The aromatic groups represented by the formulas (2a) and (2c) to (2e) may be substituted with a substituent at an arbitrary position. Examples of a substituent include an alkyl group such as a methyl group, an ethyl group, an isopropyl group, an n-propyl group, and at-butyl group; an alkoxy group such as a methoxy group, an ethoxy group, an n-propoxy group, and an n-butoxy group; a halogen atom such as a fluorine atom, a chlorine atom, and a bromine atom; and the like. Ar is preferably the aromatic group represented by the formula (2a) or (2b), and particularly preferably the aromatic group represented by the formula (2b).

X¹ to X³ are independently a divalent organic group, or a single bond that directly bonds Ar and Y¹, Y², or Y³. The divalent organic group that may be represented by X¹ to X³ is preferably a group that can form a pi electron conjugated system together with Ar. When the divalent organic group that may be represented by X¹ to X³ forms a pi electron conjugated system, the planarity of the tridentate ligand represented by the formula (1) is improved, and a strong three-dimensional network structure is easily formed. The number of carbon atoms of the divalent organic group is preferably 2 to 18, more preferably 2 to 12, and still more preferably 2 to 6.

Y¹ to Y³ are independently a monovalent organic group having a coordinating moiety. The organic group represented by Y¹ to Y³ is preferably a group that can form a pi electron conjugated system together with Ar and X¹ to X³. When the organic group represented by Y¹ to Y³ forms a pi electron conjugated system, the planarity of the tridentate ligand represented by the formula (1) is improved, and a strong three-dimensional network structure is easily formed.

The number of carbon atoms of the organic group represented by Y¹ to Y³ is preferably 5 to 11, and more preferably 5 to 7. Examples of the organic group represented by Y¹ to Y³ include the organic groups respectively represented by the following formulas (3a) to (3f). It is noted that"*" in the formulas (3a) to (3f) indicates the position at which X¹, X², or X³ is bonded.

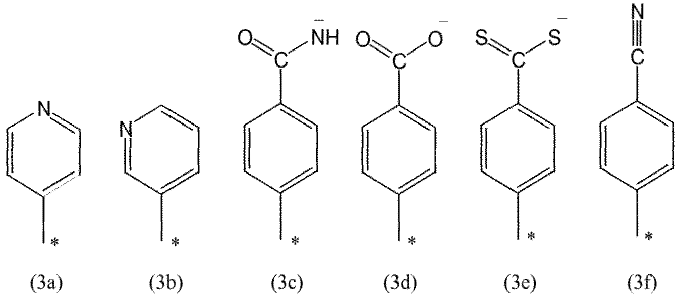

The organic groups represented by the formulas (3a) to (3f) may be substituted with a substituent at an arbitrary position. Examples of the substituent include those mentioned above in connection with Ar. The group represented by the formula (3a) is particularly preferable as Y¹ to Y³.

The size of the pores and the like of the polynuclear-metal complex can be adjusted by appropriately selecting Ar, X¹ to X³, and Y¹ to Y³ in the tridentate ligand represented by the formula (1). The method according to one embodiment of the invention makes it possible to efficiently obtain a stable single crystal of a polynuclear metal complex that has pores and the like having a size sufficient to include an organic compound for which the molecular structure is to be determined.

It is preferable that the tridentate ligand represented by the formula (1) have high planarity and high symmetry and have a structure in which a pi-conjugated system extends over the entire ligand, since a strong three-dimensional network structure is easily formed. Examples of such a tridentate ligand include the ligands respectively represented by the following formulas (4a) to (4f).

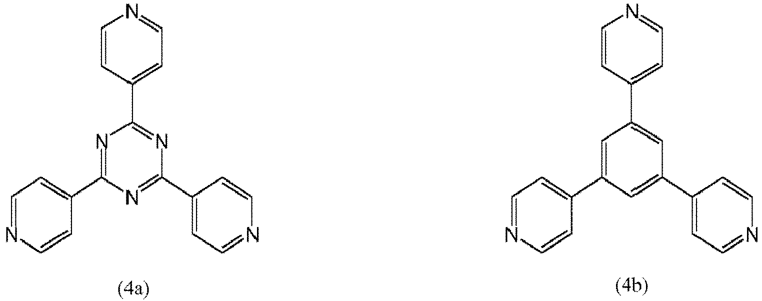

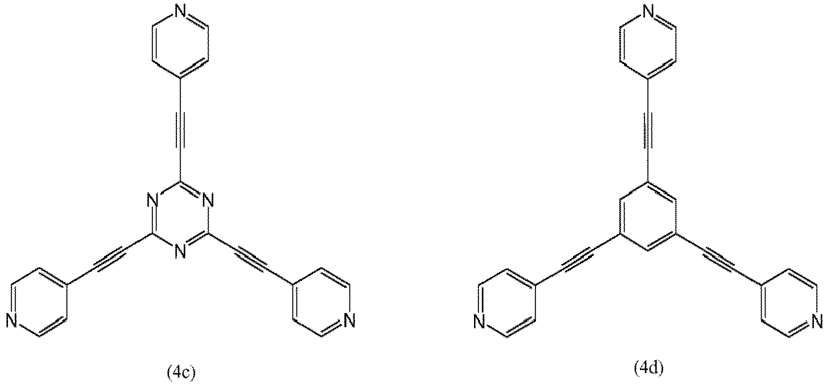

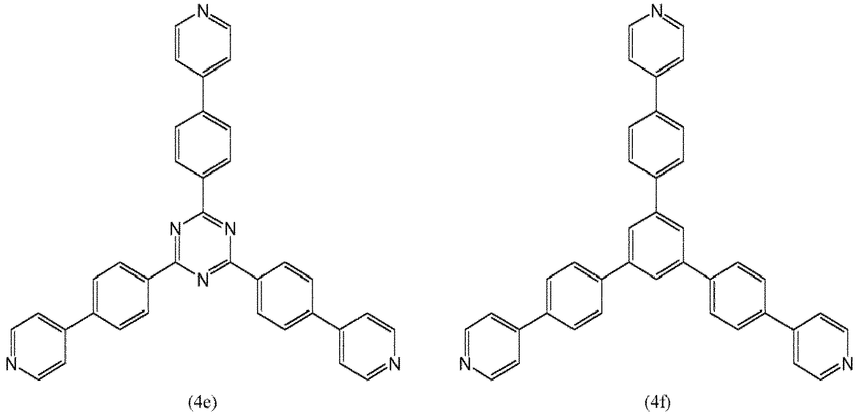

Among these, 2,4,6-tris(4-pyridyl)-1,3,5-triazine (TPT) represented by the formula (4a) is particularly preferable as the tridentate ligand represented by the formula (1).

The metal ion that serves as the center metal is not particularly limited as long as the metal ion forms a coordination bond together with the multidentate ligand to form the three-dimensional network structure. A known metal ion may be used as the metal ion that serves as the center metal. It is preferable to use an ion of a metal among the metals that belong to Groups 8 to 12 in the periodic table, such as an iron ion, a cobalt ion, a nickel ion, a copper ion, a zinc ion, or a silver ion, and more preferably an ion of a divalent metal among the metals that belong to Groups 8 to 12 in the periodic table. It is particularly preferable to use a zinc(II) ion or a cobalt(II) ion, since a polynuclear-metal complex having large pores and the like can be easily obtained. Even more preferably is the use of zinc(II) ion.

The crystalline polynuclear metal complex used in connection with the embodiments of the invention is normally stabilized due to coordination of a monodentate ligand that serves as a counter ion in addition to the neutral multidentate ligand. Examples of the monodentate ligand include a monovalent anion such as a halogen ion such as a chloride ion (Cl⁻), a bromide ion (Br⁻) and an iodide ion (I⁻), and a thiocyanate ion (SCN⁻). Preferably the monovalent anion is a chloride ion (Cl⁻).

The crystalline polynuclear metal complex used in connection with the embodiments of the invention has the three-dimensional network structure that is formed by the metal ion and the tridentate ligand that is coordinated to the metal ion, and has pores and the like which may be three-dimensionally arranged in the three-dimensional network structure in an ordered manner.

The expression "the pores and the like that are three-dimensionally arranged in the three-dimensional network structure in an ordered manner" means that the pores and the like are arranged in the three-dimensional network structure in an ordered manner to such an extent that the pores and the like can be observed by X-ray single crystal structure analysis. The three-dimensional network structure is not particularly limited as long as the three-dimensional network structure has the above structural features, and the pores and the like have a size sufficient to include the analyte guest compound, often an organic compound, for which the molecular structure is to be determined.

In a preferred embodiments of the present invention, the crystalline polynuclear metal complex includes Zn(ll) as the metal ion. In such preferred embodiments the monovalent anion is either chloride (CI-) or Iodide (I-). In such crystalline polynuclear metal complex the preferred ligand Lis 2,4,6-tris(4-pyridyl)-1,3,5-triazine (TPT).

In addition to the selection of the crystalline polynuclear metal complex for introducing the analyte guest compound, the selection of the optimal (solution) conditions ("soaking conditions") is important to achieve a sufficient amount of analyte guest compound to be included into the crystalline polynuclear metal complex. Such (solution) conditions refer for example to the selected solvent used during the period wherein the analyte guest compound containing solution is in contact with the crystalline polynuclear metal complex and the selected temperature. Furthermore, a successful selection method requires that the amount of analyte guest compound that is indeed introduced into the crystalline polynuclear metal complex is accurately determined and preferably in a rapid or relatively rapid manner.

As such the present invention is directed to a method to select conditions for introduction of an analyte in a crystalline polynuclear metal complex for use in single crystal X ray crystallography, wherein the crystalline polynuclear metal complex represented by [[M(X)₂]₃(L)₂]ₙ, wherein M is a metal ion, X is a monovalent anion, L is a tridentate ligand represented by a formula (1), 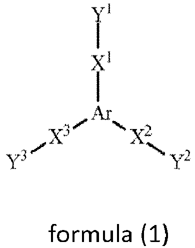 wherein Ar is a substituted or unsubstituted trivalent aromatic group, X¹ to X³ are independently a divalent organic group, or a single bond that directly bind Ar and Y¹, Y², or Y³, and Y¹ to Y³ are independently a monovalent organic group having a coordinating moiety, and n is an arbitrary natural number, the method comprising:
a) contacting a plurality of samples of the analyte with the crystalline polynuclear metal complex under a plurality of solution conditions to obtain a plurality of sample analyte polynuclear metal complexes in solution,
b) evaporating the solvent from each sample analyte polynuclear metal complex,
c) determine the amount of analyte included in each sample analyte polynuclear metal complex, and
d) selecting the solution condition resulting in the highest amount of analyte included in the sample analyte polynuclear metal complex.

In order to more rapidly screed for an optimal selected method the method as in the above described embodiment can be carried out in parallel wherein steps a) to c) of the method are carried out in parallel. This would allow for screening multiple possible soaking conditions at the same time thereby significantly reducing the process for selecting the optimal type of crystalline sponge and soaking conditions which would introduce a sufficient amount of analyte guest compound into a crystalline sponge.

In another embodiment the step c) of the above method further comprises the steps of washing the sample analyte polynuclear metal complex obtained in step b) of said method, dissolving the resulting sample analyte polynuclear metal complex and determining the amount of sample analyte (analyte guest compound) introduced into the crystalline polynuclear metal complex using a quantitative analytical method. This washing step reduces the amount of analyte guest compound that is adsorbed externally onto the crystalline polynuclear metal complex. Such analyte guest compound that is adsorbed on the external surface of the crystalline sponge is not arranged in an ordered matter and would not be useful in the structure elucidation using X-ray crystallography. When determining whether a soaking condition is successfully introducing the analyte guest compound into the crystalline polynuclear metal complex such analyte guest compound adsorbed to the external surface of the crystalline polynuclear metal complex should not be included in the analysis. Washing with an appropriate solution or washing fluid to remove such analyte guest compound adsorbed to the external surface of the crystalline sponge allows for a more accurate measurement of the amount of analyte guest compound which is introduced into the crystalline sponge material.

Any washing fluid or solution which would remove the analyte guest compound from the external surface of the crystalline polynuclear metal complex can be used. Preferably, the washing fluid or solution does not affect the crystalline sponge, or the analyte guest compound introduced into the crystalline sponge. A preferred washing fluid or solution is a dilute aqueous solution, more preferably the washing fluid is water. The washing step is carried out at a temperature from about 15°C to about 40°C, preferably at about room temperature (i.e. from about 20°C to about 30°C, or 25°C +/- 5°C) for a short period of time. Preferably this washing period is from about 1 min to about 15 min, more preferable from about 2 min to about 10 min, even more preferably from about 3 min to about 7 min.

The step of dissolving the obtained sample analyte polynuclear metal complex is intended to release the analyte guest compound (sample analyte) which has been introduced into the polynuclear metal complex during the experimental soaking conditions. Dissolving of the obtained sample analyte polynuclear metal complex includes an appropriate dissolution medium which would destroy and dissolve the crystalline structure of the crystalline polynuclear metal complex. Any dissolution medium that would dissolve the crystal structure can be used. In a preferred embodiment the dissolution medium comprises a mixture of acetone, acetonitrile, water and methanol. In a more preferred embodiment, the dissolution medium is mixture of acetone, acetonitrile, water and methanol at 25% V/V each. The step of dissolving the sample analyte crystalline polynuclear metal complex using the dissolution medium is carried out for a period of 6 to 36 hours, preferably for a period of 10 to 30 hours, more preferably for a period of 12 to 24 hours. Dissolution is carried out at a temperature from about 15°C to about 40°C, preferably at about room temperature (i.e. from about 20°C to about 30°C, or 25°C +/- 5°C).

The obtained analyte solution containing the analyte guest compound that had been successfully introduced into the crystalline polynuclear metal complex under the experimental soaking conditions is subjected to quantitative analysis. To determine the quantitative amount of analyte guest compound introduced into the crystalline sponge the materials are analyzed with a quantitative analytical method selected from LC-MS/MS, LC-UV, LC-PDA (Photodiode array-Detection), LC-RF (fluorescence detection), LC-RI (refractive index detection), LC-ELSD (light scattering detection), LC-CCD (conductivity detection. The reference to LC here refers to liquid chromatography, as such liquid chromatography includes any type of liquid chromatography such as HPLC and UPLC.

The experimental soaking conditions to be screened in the selection method of the present invention include, in addition to the selection of the type of crystalline sponge, the type of solvent and temperature conditions for soaking the analyte guest compound into the crystalline polynuclear metal complex. Accordingly, the method of the present invention includes a screening of a plurality of (solution) conditions (i.e. soaking conditions) which includes a plurality of temperature conditions and/or a plurality of solvents. Preferably the experimental solvent is any different solvent used in combination with a particular type of crystalline polynuclear metal complex (crystalline sponge). Such solvents is selected from acyclic and cyclic alkanes, alkenes, ethers, alcohols, ketones, dichloromethane, trichloromethane, esters, dimethylformamide, dimethylsulfoxide, tetrahydrofuran (THF) and mixtures thereof. With respect to the experimental temperature conditions to be evaluated in the screening method these vary also depending on the particular crystalline polynuclear metal complex and can include a series of experimental temperatures for "soaking" the analyte guest compound into the crystalline polynuclear metal complex. Such temperature can be selected from for example 4°C, 25°C and 50°C or any other suitable soaking temperature.

In addition, the above methods the visual inspection of the crystalline sponge under each experimental soaking condition can be used as a criterium to selected the optimal soaking conditions for introducing the analyte guest compound into the crystalline polynuclear metal complex.

Further, the selection method described herein is useful not only to determine the optimal soaking conditions for a single analyte guest compound but could also be used in a single screening to determine the optimal soaking conditions for each guest analyte compound in a mixture of guest analyte compounds. In such a method the determination step in any of the embodiments described herein would not only include a quantitative analytical method but also a qualitative analytical method. In some embodiments of such method the quantitative and qualitative analytical method can be the same analytical method such as for example an analytical method selected from LC-MS/MS, LC-UV, LC-PDA (Photodiode array-Detection), LC-RF (fluorescence detection), LC-RI (refractive index detection), LC-ELSD (light scattering detection), LC-CCD (conductivity detection. In such a method of the present invention the selection of optimal soaking conditions for each analyte guest compound in a mixture can be determined based when a plurality of soaking conditions are screened simultaneously for the mixture of analyte guest compounds.

In another embodiment of the present invention there is provided a method of introducing an analyte into a crystalline polynuclear metal complex for use in single crystal X ray crystallography, wherein the crystalline polynuclear metal complex represented by [[M(X)₂]₃(L)₂]ₙ, wherein M is a metal ion, X is a monovalent anion, L is a tridentate ligand represented by a formula (1), 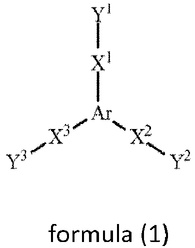 wherein Ar is a substituted or unsubstituted trivalent aromatic group, X¹ to X³ are independently a divalent organic group, or a single bond that directly bind Ar and Y¹, Y², or Y³, and Y¹ to Y³ are independently a monovalent organic group having a coordinating moiety, and n is an arbitrary natural number, the method comprising:
a. selecting the solution conditions for introducing the analyte into the crystalline polynuclear metal complex using the method as described in any of the above embodiments, and
b. contacting the analyte and crystalline polynuclear metal complex at the solution conditions selected in step a) to obtain an analyte crystalline polynuclear metal complex.

In yet another embodiment the present invention provides a method of determining the crystal structure of an analyte through single crystal X ray crystallography using an analyte polynuclear metal complex obtained using the method as in any of the embodiments described above. In Figure 1 a representation is provided for such method wherein a selection is made for the optimal soaking conditions followed by the use of such soaking conditions to obtain an appropriate amount of analyte guest compound introduced into the crystalline sponge for use in a structure elucidation using X-ray crystallography.

### Examples

### Example 1: Selection of optimal soaking conditions for dehydronifedipine.

The compound dehydronifedipine was soaked and processed as follows. The simultaneous soaking of 1000 ng dehydronifedipine (1mg/mL in dichloromethane) was conducted with both crystalline sponge types (ZnCl₂ and ZnI₂), in two different storage solvents (cyclohexane and n-hexane) at two different temperatures (25°C and 50°C) over 24 hours. Following this the obtained material was washed with water and crystal size measurement was carried out. Subsequently the crystalline sponge complexes were dissolved in the dissolution medium. After which quantitation was carried out of soaked analytes to compare the different soaking conditions to one and other. The quantitation results plus the microscopic observation of the crystals (crystallinity/ dampness/color) gave a clear prioritization order for the preparation of further XRD experiments. The top condition is ZnI₂ in cyclo-hexane at 50°C followed by ZnI₂ in cyclohexane at 25°C and ZnCl₂ in n-hexane at 50°C/ and ZnCl₂ in n-hexane at 50°C. The XRD results underline that the top priority shows better results than a lower ranked soaking conditions. The structural determination of dehydronifedipine in a ZnI₂ sponge with cyclo-hexane as soaking solvent and a soaking temperature at 50°C was successful. No analyte was assignable for ZnI₂ in cyclohexane at 25°C and ZnCl₂ in n-hexane sponges at 50°C were broken after the soaking and in the ZnCI2 in cyclo-hexane sponges at 50°C no dehydronifedipine or solvent (cyclo-hexane) was assignable through XRD.

As such the method provided a selection method to select the optimal soaking conditions to introduce a sufficient amount of the analyte into the crystalline sponge as confirmed by a successful structure elucidate by X-ray crystallography.

## Claims

1. A method to select conditions for inclusion of an analyte in a crystalline polynuclear metal complex for use in single crystal X ray crystallography, wherein the crystalline polynuclear metal complex represented by [[M(X)₂]₃(L)₂]ₙ, wherein M is a metal ion, X is a monovalent anion, L is a tridentate ligand represented by a formula (1), 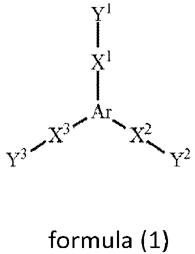 wherein Ar is a substituted or unsubstituted trivalent aromatic group, X¹ to X³ are independently a divalent organic group, or a single bond that directly bind Ar and Y¹, Y², or Y³, and Y¹ to Y³ are independently a monovalent organic group having a coordinating moiety, and n is an arbitrary natural number, the method comprising:
a) contacting a plurality of samples of the analyte with the crystalline polynuclear metal complex under a plurality of solution conditions to obtain a plurality of sample analyte polynuclear metal complexes in solution,
b) evaporating the solvent from each sample analyte polynuclear metal complex,
c) determine the amount of analyte included in each sample analyte polynuclear metal complex, and
d) selecting the solution condition resulting in the highest amount of analyte included in the sample analyte polynuclear metal complex.

2. The method of claim 1, wherein steps a) to c) are carried out in parallel.

3. The method of claim 1 or 2, wherein step c) comprises
i. washing the sample analyte polynuclear metal complex obtained in step b),
ii. dissolving the sample analyte polynuclear metal complex obtained in the previous step, and
iii. determine the amount of sample analyte introduced into the crystalline polynuclear metal complex using a quantitative analytical method.

4. The method of any preceding claim, wherein the amount of analyte included in each sample analyte polynuclear metal complex is determined using a quantitative analytical method selected from LC-MS/MS, LC-UV, LC-PDA (Photodiode array-Detection), LC-RF (fluorescence detection), LC-RI (refractive index detection), LC-ELSD (light scattering detection), and LC-CCD (conductivity detection).

5. The method of any preceding claim, wherein the plurality of solution conditions comprises selecting a plurality of temperature conditions and/or a plurality of solvents.

6. The method of claim 5, wherein the plurality of temperature conditions comprises contacting the plurality of samples of the analyte with the crystalline polynuclear metal complex in solution at two or more temperatures selected from 4°C, 25°C and 50°C.

7. The method of claim 5 or 6, wherein the solvent is selected from acyclic or cyclic alkenes, ethers, alcohols, ketones, dichloromethane, trichloromethane, esters, dimethylformamide, dimethylsulfoxide, tetrahydrofuran and mixtures thereof.

8. A method of introducing an analyte into a crystalline polynuclear metal complex for use in single crystal X ray crystallography, wherein the crystalline polynuclear metal complex represented by [[M(X)₂]₃(L)₂]ₙ, wherein M is a metal ion, X is a monovalent anion, L is a tridentate ligand represented by a formula (1), 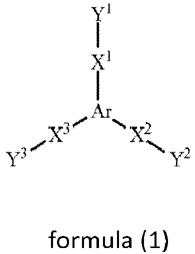 wherein Ar is a substituted or unsubstituted trivalent aromatic group, X¹ to X³ are independently a divalent organic group, or a single bond that directly bind Ar and Y¹, Y², or Y³, and Y¹ to Y³ are independently a monovalent organic group having a coordinating moiety, and n is an arbitrary natural number, the method comprising:
a. selecting the solution conditions for introducing the analyte into the crystalline polynuclear metal complex using the method according to any of the preceding claims, and
b. contacting the analyte and crystalline polynuclear metal complex at the solution conditions selected in step a) to obtain an analyte crystalline polynuclear metal complex.

9. A method of determining the crystal structure of an analyte through single crystal X-ray crystallography using an analyte polynuclear metal complex obtained using the method of claim 8.
